# EUROPEAN PATENT SPECIFICATION

(11) **EP 1 416 901 B1**
(45) Date of publication and mention of the grant of the patent: **26.10.2005**
(21) Application number: 02756998.7
(22) Date of filing: 06.08.2002
(51) Int. Cl.: A61K 6/00, A61K 6/087

(54) **DENTAL ROOT CANAL FILLING CONES**
KEGEL ZUR FÜLLUNG VON ZAHNWURZELKANÄLEN
CONES D'OBTURATION DE CANAUX RADICULAIRES

(30) Priority: 13.08.2001 US 312017 P; 06.08.2002 US 213320
(43) Date of publication of application: 12.05.2004
(73) Proprietor: DENTSPLY INTERNATIONAL, INC., York, PA 17405-0872 (US)
(72) Inventor: KLEE, Joachim, E., 78315 Radolfzell (DE)
(74) Representative: Hartz, Nikolai
(86) International application number: PCT/US2002/025004
(87) International publication number: WO 2003/015718

(56) References cited:
- EP-A- 0 608 361
- US-A- 5 624 976
- HUANG T-H ET AL: "The biocompatibility evaluation of epoxy resin-based root canal sealers in vitro" BIOMATERIALS, ELSEVIER SCIENCE PUBLISHERS BV., BARKING, GB, vol. 23, no. 1, 1 January 2002 (2002-01-01), pages 77-83, XP004322622 ISSN: 0142-9612

## Description

### TECHNICAL FIELD

Described are dental root canal filling cones comprising: filler and thermoplastic polymer, wherein said thermoplastic polymer is formed by polymerization of polymerizable diepoxide monomer and amine monomer, said amine monomers being primary monoamine and/or a disecondary diamine, said filler comprising 40 to 90 weight-% of said cones providing a radio-opacity of at least 3 mm/mm aluminum.

### BACKGROUND OF THE INVENTION

In the last decades gutta-percha cones in combination with a root canal sealer are the most popular material used for root canal filling by master-point technique or by lateral condensation. The clinical success of a root canal filling depends on complete and tight filling. To improve the clinical success further and to make the root canal therapy more easily and safe both an excellent connection between root canal sealer and cavity wall on the one side and canal sealer and root canal cones must be achieved by new application technique.

Due to the polar hydrophilic moieties epoxide-amine basing root canal sealer adapt well to the cavity walls. Proofed is the tightness of a filled root canal by numerous studies. Moreover, recently, an adhesion of the epoxide-amine basing root canal sealer AH Plus (Dentsply De Trey) of 4 MPa was measured (JD Pécora et al., Braz.Dent.J. **12** (2001) 27) that shows how well the material is bonded to the cavity walls.

It is well-known that polymers of different polymer classes frequently are thermodynamically incompatible. That means they do not undergo any connection and they do not adhere well to each other. Same is the case for non-polar gutta-percha and the most of the more polar root canal filling materials. Consequently, a demand is to make root canal cones and root canal sealer more compatible.

In view of the further treatment, root canal sealer as well as root canal cones shell be thermoplastic to be removable if corrections are demanded or core build-up shell occur.

Recently, the first thermoplastic root canal sealer was invented (US 5624976, 25.03.1994).

Recently, a thermosetting resin based material was applied for thermoset resin cones whereby either the root canal sealer or the cone are conductive ones (EP 0608361). There are some disadvantages for thermosetting cones, because they are:
- difficult to remove
- difficult to cut
- not re-workable by thermal or solution processes
- not suitable for injection molding, casting processes or related processes
- not suitable for special filler treatment as described by this patent.

It is an object of the invention to provide a soluble and thermoplastic dental root canal cone that is easy to remove, that undergoes a connection to the thermoplastic sealer and which provides a radio-opacity of at least 3 mm/mm Al.

### DESCRIPTION OF THE PREFERRED EMBODIMENTS

Invented dental root canal filling cones comprise: filler and thermoplastic polymer, wherein said thermoplastic polymer is formed by polymerization of polymerizable diepoxide monomer and amine monomer, said amine monomers being primary monoamine and/or a disecondary diamine, said filler comprising 40 to 90 weight-% of said cones providing a radio-opacity of at least 3 mm/mm aluminum.

The dental root canal filling cones are composed of at least one thermoplastic polymer or they are composed of a thermoplastic polymer in the outer sphere of the cone and a core material in the inner sphere selected from the group of metals, ceramics, glass fibers or other thermoplastic or thermosetting plastic polymers such as polyamides, polyester, polyurethanes, polyethylene or polypropylene.

The thermoplastic polymers of the dental root canal filling cones are selected from the group of epoxide-amine addition polymers of the general formulas: 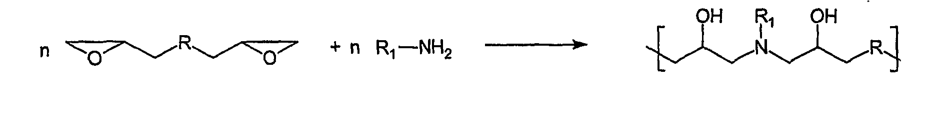 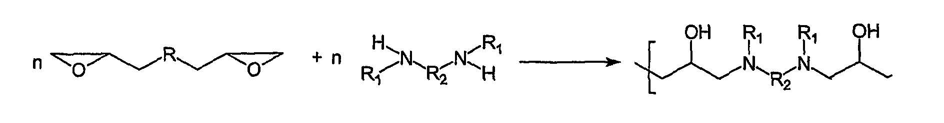 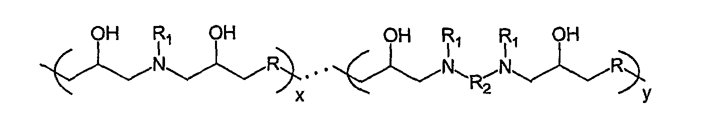 wherein R is a moiety formed from a diepoxide, such as 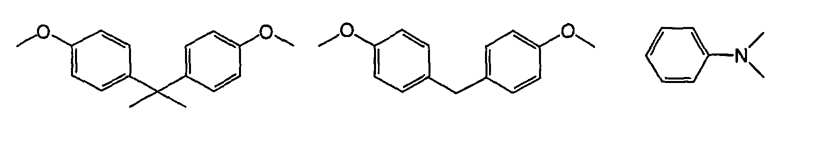 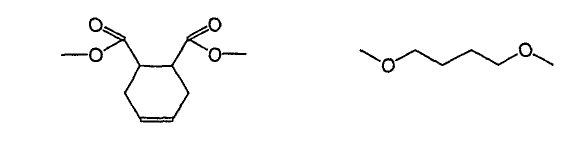 R₁ denotes a monofunctional substituted C₁ to C₁₈ alkylene, a substituted or unsubstituted C₅ to C₁₈ cycloalkylene, a substituted or unsubstituted C₅ to C₁₈ arylene or heteroarylene, such as 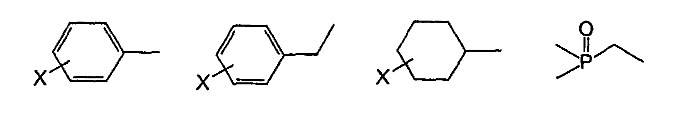 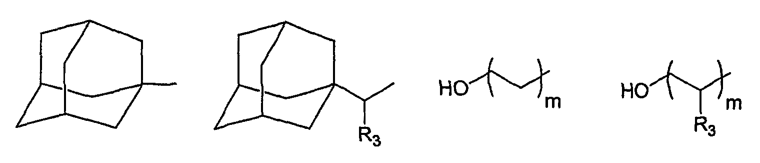 R₂ denotes a difunctional substituted or unsubstituted C₁ to C₁₈ alkylene, a substituted or unsubstituted C₅ to C₁₈ cycloalkylene, a substituted or unsubstituted C₅ to C₁₈ arylene or heteroarylene, such as 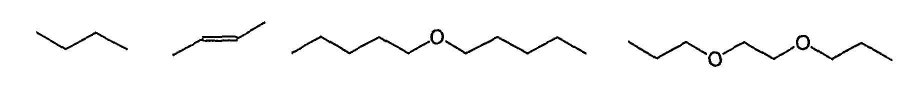 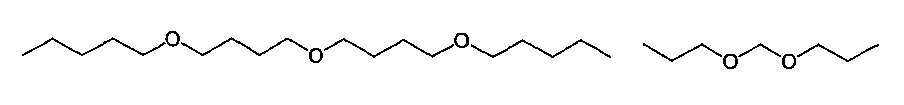 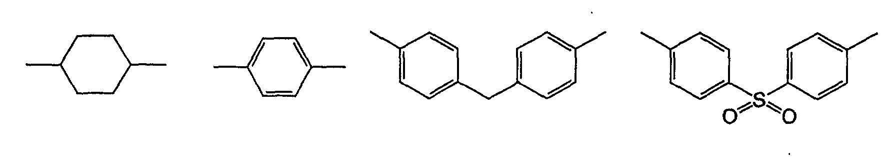 R₃ denotes hydrogen or C₁ to C₁₈ alkylene, such as H, CH₃, C₂H₅, C₃H₇ and X is hydrogen or a substituent selected from the group of OCH₃, F, Cl, Br, I, CH₃, COCH₃, NO₂, COOC₂H₅.

As epoxide monomer is used a diepoxide selected from the group of diglycidylethers such as diglycidyl ether of bisphenol-A, diglycidyl ether of bis-phenol-F, butandiol diglycidyl ether, N,N-diglycidylaniline or Δ³-tetrahydrophthalic acid diglycidyl ester.

Preferred amines are primary monoamines such as benzylamine, 1-aminoadamantane, α-phenethylamine and ethanol amine and disecondary diamines such as N,N'-dibenzyl ethylene diamine, N,N'-dibenzyl-3,6-dioxaoctanediamine-1,8, N,N'-dibenzyl-5-oxanonane diamine-1,9, N,N'-dibenzyl-(2,2,4)/(2,4,4)-trimethylhexamethylene diamine, N,N'-dicyclohexyl ethylene diamine, N,N'-dimethyl-p-xylylene diamine.

The achieve excellent mechanical properties and a high level of radio-opacity the dental root canal filling cones contains fillers such as inorganic compounds like La₂O₃, ZrO₂, BiPO₄, CaWO₄, BaWO₄, SrF₂, Bi₂O₃ or organic fillers, such as polymer granulate, splinter polymers or a combination of organic and/or inorganic fillers. Consequently, the Dental root canal filling cones provide a radio-opacity of at least 3 mm/mm Al, preferably at least 5 to 7 mm/mm Al, most preferably at least 7 mm/mm Al.

The process of preparation of dental root canal filling cones occurs by thermal addition polymerization of the diepoxide monomer and the amine monomer in presence of fillers and a simultaneous or a subsequent forming (casting) process of cones.

An other alternative is a two step-procedure, namely
i) thermal addition polymerization of the diepoxide monomer and the amine monomer on the filler surface
ii) forming (casting) process of the surface-modified filler of (i) by thermal and/or pressure processes.

### Example 1

128.313 g (337.67 mmol) bisphenol-A diglycidyl ether (Mₙ 380 g/mol), 10.535 g (33.77 mmol) bisphenol-F diglycidyl ether, 28.140 g (185.72 mmol) 1-amino-adamantane, 63.241 g (185.72 mmol) N,N'-dibenzyl-5-oxanonanediamine-1.9 and 660.070 g CaWO₄, 165.018 g ZrO₂, and 9.980 g Aerosil 200 were mixed homogeneously and polymerized 24 hours at 60 °C.

The composition is characterized by following values: radio-opacity RO= 10.1 mm/mm Al glass transition temperature T_{g} = 64 °C and volumetric shrinkage 1.13 vol.-%.

The obtained thermoplastic composite material was used for a thermal reforming process to form root canal cones.

### Example 2

250.00 g (734.39 mmol) bisphenol-A diglycidyl ether, 22.255 g (146.88 mmol) 1-amino-adamantane, 200.059 g (587.51 mmol) N,N'-dibenzyl-5-oxanonanediamine-1.9 and 2249.112 g of a Barium-alumo silicate glass were mixed homogeneously and polymerized 24 hours at 60 °C.

The composition is characterized by following values: radio-opacity RO= 3.1 mm/mm Al, glass transition temperature T_{g} = 37 °C and volumetric shrinkage 1.46 vol.-%.

## Claims

1. Dental root canal filling cones comprising: filler and thermoplastic polymer, wherein said thermoplastic polymer is obtainable by polymerization of polymerizable diepoxide monomer and amine monomer, said amine monomers being primary monoamine and/or a disecondary diamine, said filler comprising 40 to 90 weight-% of said cones providing a radio-opacity of at least 3 mm/mm aluminum.

2. Dental root canal filling cones of claim 1, composed of at least a thermoplastic polymer.

3. Dental root canal filling cones of claim 1, composed of a thermoplastic polymer in the outer sphere of the cone and a core material in the inner sphere selected from the group of metals, ceramics, glass fibers or other thermoplastic or thermosetting polymers such as polyamides, polyester, polyurethanes, polyethylene or polypropylene.

4. Dental root canal filling cones of claim 1, wherein said amine monomer and said epoxide monomer are polymerized to form polymer within the scope of at least one of the general formulas: 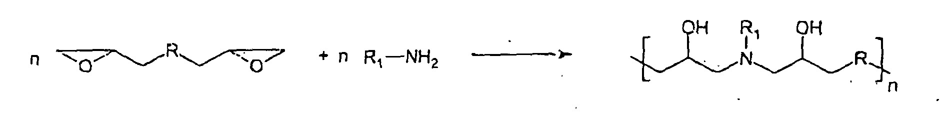 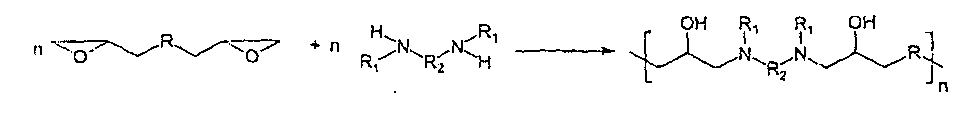 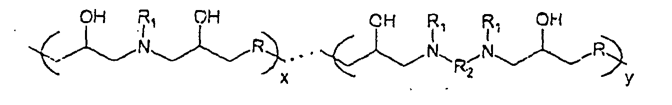 wherein R is a moiety formed from a diepoxide, selected from the group consisting of 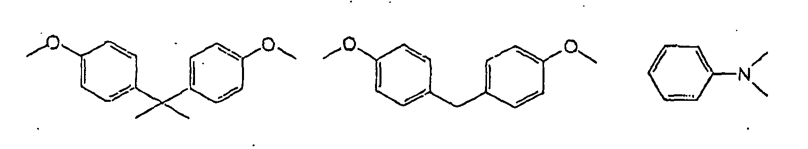 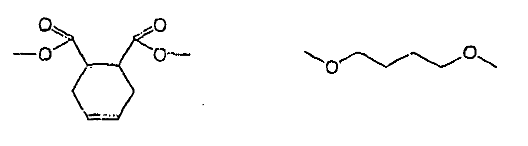 R₁ denotes a monofunctional substituted C₁ to C₁₈ alkylene, a substituted or unsubstituted C₅ to C₁₈ cycloalkylene, a substituted or unsubstituted C₅ to C₁₈ arylene or heteroarylene, selected from the group consisting of 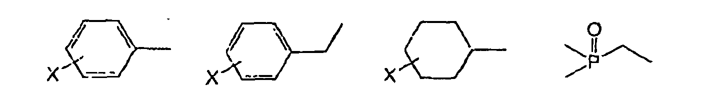 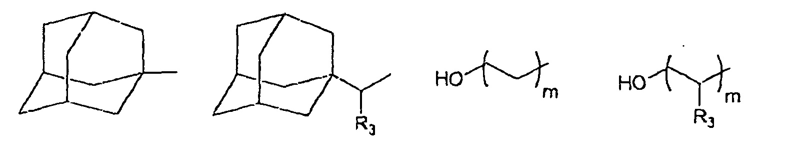 R₂ denotes a difunctional substituted or unsubstituted C₁ to C₁₈ alkylene, a substituted or unsubstituted C₅ to C₁₈ cycloalkylene, a substituted or unsubstituted C₅ to C₁₈ arylene or heteroarylene, selected from the group consisting of 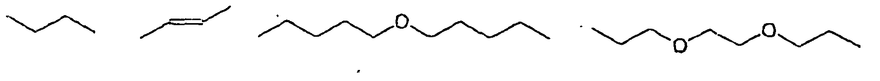 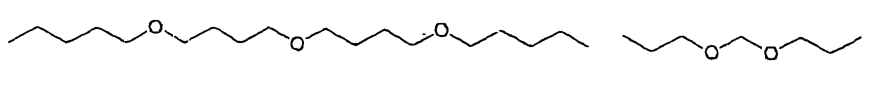 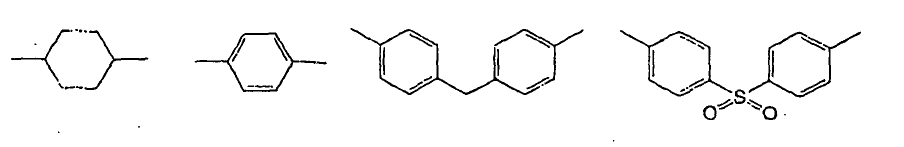 R₃ denotes hydrogen or C₁ to C₁₈ alkylene, such as H, CH₃, C₂H₅, C₃H, and
X is hydrogen or a substituent selected from the group consisting of OCH₃, F, Cl, Br, I, CH₃, COCH₃, NO₂, COOC₂H₅.

5. Dental root canal filling cones of claim 1, wherein said epoxide monomer is a diepoxide selected from the group of diglycidylethers such as diglycidyl ether of bisphenol-A, diglycidyl ether of bis-phenol-F, butandiol diglycidyl ether, N,N-diglycidylaniline or Δ³-tetrahydrophthalic acid diglycidyl ester.

6. Dental root canal filling cones of claim 1, wherein said primary monoamine preferably is benzylamine, 1-aminoadamantane, α-phenethylamine and ethanol amine.

7. Dental root canal filling cones of claim 1, wherein said disecondary diamine preferably is N,N'-dibenzyl ethylene diamine, N,N'-dibenzyl-3,6-dioxa-octanediamine-1,8, N,N'-dibenzyl-5-oxanonane diamine-1,9, N,N'-dibenzyl-(2,2,4)/(2,4,4)-trimethylhexamethylene diamine, N,N'-dicyclohexyl ethylene diamine, N,N'-dimethyl-p-xylylene diamine.

8. Dental root canal filling cones of claim 1, wherein said filler is an inorganic compound such as La₂O₃, ZrO₂, BiPO₄, CaWO₄, BaWO₄, SrF₂, Bi₂O₃ or organic fillers, such as polymer granulate, splinter polymers or a combination of organic and/or inorganic fillers.

9. Dental root canal filling cones of claim 1, containing fillers which provide a radio-opacity of at least 3 mm/mm Al, preferably at least 5 to 7 mm/mm Al, most preferably at least 7 mm/mm Al.

10. Dental root canal filling cones of claim 1, wherein said cones are soluble in polar organic solvents such as CHCl₃, tetrahydrofuran or dimethyl formamide, said cones producible from those polymer solutions.

11. Dental root canal filling cones of claim 1, wherein said cones contain additives such as stabilizer and plasticizer.

12. Process for the preparation of dental root canal filling cones of claim 1, comprising: filler and thermoplastic polymer, wherein said thermoplastic polymer is formed by polymerization of polymerizable diepoxide monomer and amine monomer, said amine monomers being primary monoamine or a disecondary diamine, said filler comprising 40 to 90 weight-% of said cones providing a radio-opacity of at least 3 mm/mm aluminum **characterized by** a thermal addition polymerization of the diepoxide monomer and the amine monomer and a simultaneous or a subsequent thermally workable or processes.

13. Process according to claim 12, comprising the steps of
i) thermal addition polymerization of the diepoxide monomer and the amine monomer on the filler surface; and
ii) forming (casting) process of the surface-modified filler of (i) by thermal and/or pressure processes.

## Patentansprüche

1. Konus zur Füllung von Zahnwurzelkanälen, der aufweist: einen Füllstoff und ein thermoplastisches Polymer, worin das thermoplastische Polymer erhältlich ist durch Polymerisation eines polymerisierbaren Diepoxid-Monomers und eines Amin-Monomers, wobei die Amin-Monomere primäre und/oder disekundäre Diamine sind, der Füllstoff 40 bis 90 Gew.-% des Konus umfasst, der eine Radio-Opazität von mindestens 3 mm/mm Aluminium ergibt.

2. Konus zur Füllung von Zahnwurzelkanälen nach Anspruch 1, zusammengesetzt aus mindestens einem thermoplastischen Polymer.

3. Konus zur Füllung von Zahnwurzelkanälen nach Anspruch 1, zusammengesetzt aus einem thermoplastischen Polymer am Außenbereich des Konus und einem Kernmaterial im Innenbereich, ausgewählt aus der Gruppe von Metallen, Keramik, Glasfasern oder anderen thermoplastischen oder wärmehärtenden Polymeren, wie zum Beispiel Polyamiden, Polyester, Polyurethanen, Polyethylen oder Polypropylen.

4. Konus zur Füllung von Zahnwurzelkanälen nach Anspruch 1, worin das Amin-Monomer und das Epoxid-Monomer so polymerisiert werden, dass sie ein Polymer im Rahmen von mindestens einer der allgemeinen Formeln bilden. 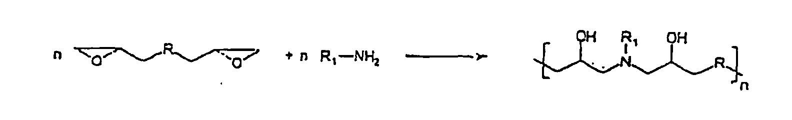 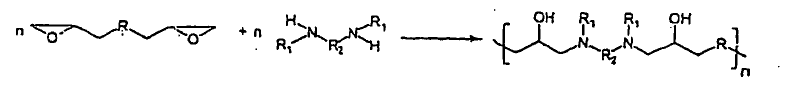 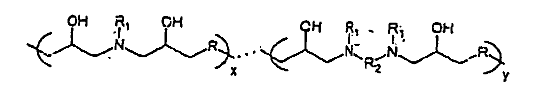 worin R eine aus einem Diepoxid gebildete Einheit ist, ausgewählt aus der Gruppe bestehend aus 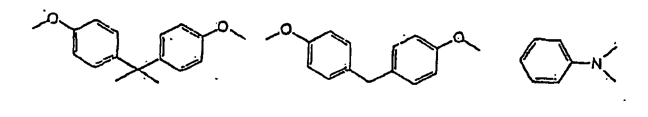 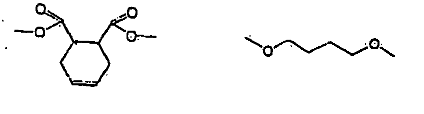 R₁ ein monofunktionelles, substituiertes C₁ bis C₁₈-Alkylen, ein substituiertes oder unsubstituiertes C₅ bis C₁₈-Cycloalkylen, ein substituiertes oder unsubstituiertes C₅ bis C₁₈-Arylen oder -Heteroarylen bedeutet, ausgewählt aus der Gruppe bestehend aus 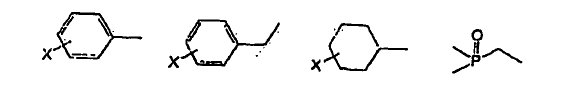 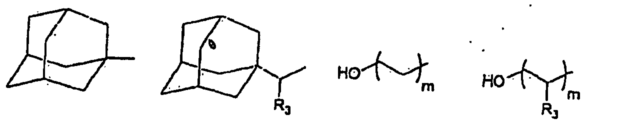 R₂ ein difunktionelles, substituiertes oder unsubstituiertes C₁ bis C₁₈-Alkylen, ein substituiertes oder unsubstituiertes C₅ bis C₁₈-Cycloalkylen, ein substituiertes oder unsubstituiertes C₅ bis C₁₈-Arylen oder -Heteroarylen bedeutet, ausgewählt aus der Gruppe bestehend aus 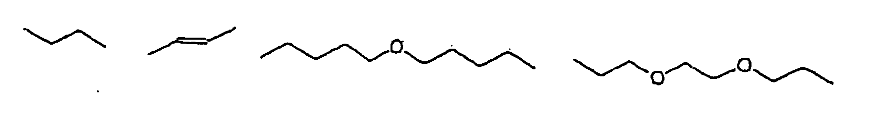 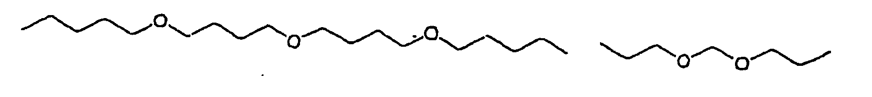 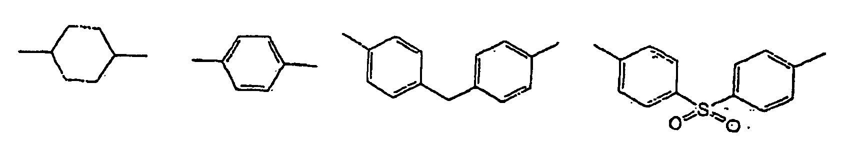 R₃ Wasserstoff oder C₁ bis C₁₈-Alkylen bedeutet, wie zum Beispiel H, CH₃, C₃H₅, C₃H₇, und
X Wasserstoff oder ein Substituent ist, ausgewählt aus der Gruppe bestehend aus OCH₃, F, Cl, Br, J, CH₃, COCH₃, NO₂, COOC₂H₅.

5. Konus zur Füllung von Zahnwurzelkanälen nach Anspruch 1, worin das Epoxid-Monomer ein Diepoxid ist, ausgewählt aus der Gruppe von Diglycidylethern, wie zum Beispiel Diglycidylether von Bisphenol-A, Diglycidylether von Bisphenol-F, Butandioldiglycidylether, N,N-Diglycidylanilin oder Δ³-Tetrahydrophthalsäurediglycidylester.

6. Konus zur Füllung von Zahnwurzelkanälen nach Anspruch 1, worin das primäre Monoamin vorzugsweise Benzylamin, 1-Aminoadamantan, α-Phenylethylamin und Ethanolamin ist.

7. Konus zur Füllung von Zahnwurzelkanälen nach Anspruch 1, worin das disekundäre Diamin vorzugsweise N,N'-Dibenzylethylendiamin, N,N'-Dibenzyl-3,6-dioxa-octandiamin-1,8, N,N'-Di-benzyl-5-oxanonandiamin-1,9, N,N'-Dibenzyl-(2,2,4)/(2,4,4)-trimethylhexamethylendiamin, N,N'-Dicyclohexylethylendiamin, N,N'-Dimethyl-p-xylylendiamin ist.

8. Konus zur Füllung von Zahnwurzelkanälen nach Anspruch 1, worin der Füller eine anorganische Verbindung, wie zum Beispiel La₂O₃, ZrO₃, BiPO₄, CaWO₄, BaWO₄, SrF₂, Bi₂O₃ oder ein organischer Füller ist, wie zum Beispiel Polymergranulate, Spanpolymere oder eine Kombination von organischen und/oder anorganischen Füllern ist.

9. Konus zur Füllung von Zahnwurzelkanälen nach Anspruch 1, der Füller aufweist, die eine Radio-Opazität von mindestens 3 mm/mm Aluminium, vorzugsweise mindestens 5 bis 7 mm/mm Aluminium, insbesondere mindestens 7 mm/mm Aluminium, ergeben.

10. Konus zur Füllung von Zahnwurzelkanälen nach Anspruch 1, worin der Konus in polaren, organischen Lösungsmitteln, wie zum Beispiel CHCl₃, Tetrahydrofuran oder Dimethylformamid löslich ist, und der Konus aus diesen Polymerlösungen herstellbar ist.

11. Konus zur Füllung von Zahnwurzelkanälen nach Anspruch 1, worin der Konus Additive, wie zum Beispiel Stabilisatoren und Weichmacher, enthält.

12. Verfahren zur Herstellung eines Konus zur Füllung von Zahnwurzelkanälen nach Anspruch 1, umfassend: Füller und thermoplastisches Polymer, wobei das thermoplastische Polymer durch Polymerisation eines polymerisierbaren Diepoxid-Monomers und eines Amin-Monomers gebildet wird, die Amin-Monomeren ein primäres Monoamin oder ein disekundäres Diamin ist, der Füllstoff 40 bis 60 Gew.-% des Konus umfasst und eine Radio-Opazität von mindestens 3 mm/mm Aluminium ergibt, **gekennzeichnet durch** eine thermische Additionspolymerisation des Diepoxid-Monomers und des Amin-Monomers und eine gleichzeitige oder aufeinander folgende thermische Verarbeitung oder Prozess.

13. Verfahren nach Anspruch 12, das die Stufen umfasst:
(i) thermische Additionspolymerisation des Diepoxid-Monomers und des Amin-Monomers auf der Füller-Oberfläche; und
(ii) Bildungs (Formgebungs)-Verfahren des Oberflächenmodifizierten Füllers von (i) durch thermische und/oder Druckverfahren.

## Revendications

1. Cônes d'obturation du canal radiculaire dentaire, comprenant : une charge et un polymère thermoplastique, dans lesquels ledit polymère thermoplastique peut être obtenu par polymérisation d'un monomère diépoxyde polymérisable et d'un monomère amine, ledit monomère amine étant un monomère monoamine primaire et/ou un monomère diamine disecondaire, ladite charge représentant 40 à 90 % en poids desdits cônes possédant une radio-opacité d'au moins 3 mm/mm d'aluminium.

2. Cônes d'obturation du canal radiculaire dentaire suivant la revendication 1, constitués d'au moins un polymère thermoplastique.

3. Cônes d'obturation du canal radiculaire dentaire suivant la revendication 1, constitués d'un polymère thermoplastique dans la sphère extérieure du cône et d'une matière de noyau dans la sphère intérieure choisie dans le groupe constitué de métaux; de matières céramiques, de fibres de verre ou d'autres polymères thermoplastiques ou themmodurcissablee tels que des polyamides, un polyester, un polyuréthanne, le polyéthylène ou le polypropylène.

4. Cônes d'obturation du canal radiculaire dentaire suivant la revendication 1, dans lesquels ledit monomère amine et ledit monomère époxyde sont polymérisés pour former un polymère dans le cadre d'au moins une des formules générales : 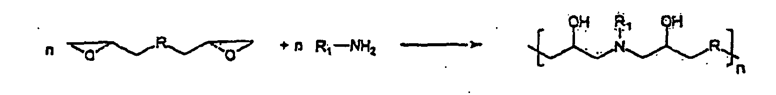 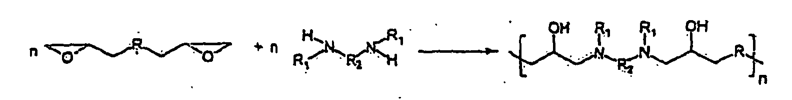 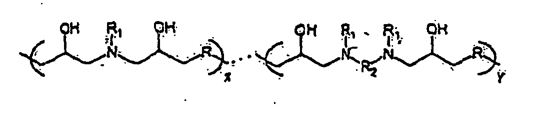 dans lesquelles R représente un groupemant formé à partir d'un diépoxyde, choisi dans le groupe consistant en : 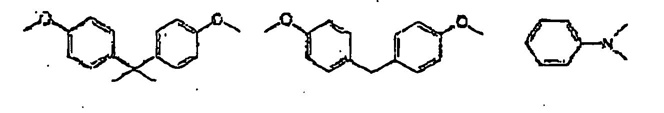 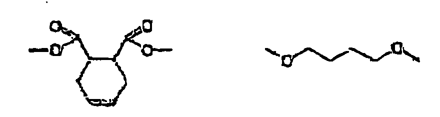 R₁ représente un groupe alkylène en C₁ à C₁₈ monofonctionnel substitué, un groupe cycloalkylène en C₅ à C₁₈ substitué ou non substitué, un groupe arylène ou hétéroarylène en C₅ à C₁₈, substitué ou non substitué, choisi dans le groupe consistant en 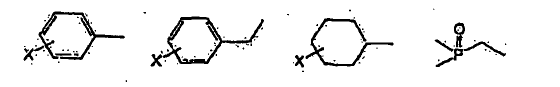 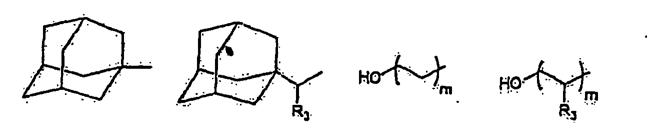 R₂ représente un groupe alkylène en C₁ à C₁₈ difonctionnel, substitué ou non substitué, un groupe cycloalkylène en C₅ à C₁₈ substitué ou non substitué, un groupe arylène ou hétéroarylène en C₅ à C₁₈, substitué ou non substitué, choisi dans le groupe consistant en 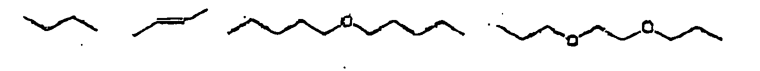 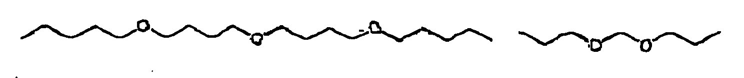 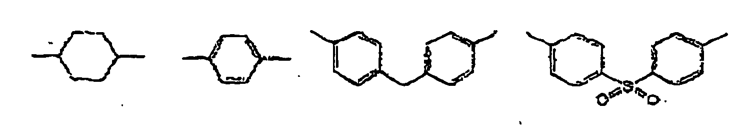 R₃ représente un atome d'hydrogène ou un groupe alkylène en C₁ à C₁₈, tel que H, CH₃, C₂H₅, C₃H, et
X représente un atome d'hydrogène ou un substituant choisi dans le groupe consistant en les substituants OCH₃, F, Cl, Br, I, CH₃ , COCH₃, NO₂, COOC₂H₅.

5. Cônes d'obturation du canal radiculaire dentaire suivant la revendication 1, dans lesquels ledit monomère époxyde est un diépoxyde choisi dans le groupe des éthers de diglycidyle tels que l'éther de diglycidyle de bisphénol-A. l'éther de diglycidyle de bisphénol-F, l'éther de diglycidyle de butanediol, la N,N-diglycidylaniline ou l'ester de diglycidyle d'acide Δ³-tétrahydrophthalique.

6. Cônes d'obturation du canal radiculaire dentaire suivant la revendication 1, dans lesquels ladite monoamine primaire est de préférence la benzylamine, le 1-aminoadamentane, l'α-phénéthylamine ou l'éthanolamine.

7. Cônes d'obturation du canal radiculaire dentaire suivant la revendication 1, dans lesquels ladite diamine disecondaire est de préférence la N,N'-dibenzyl-éthylène-diamine, la N,N'-dibenzyl-3,6-dioxa-octanediamine-1,8, la N,N'-dibenzyl-5-oxanonane-diamina-1,9, la N,N'-dibenzyl-(2,2,4)/-(2,4,4)-triméthylhexaméthylène-diamine, la N,N'-dicyclohexyl-éthylène-diamine ou la N,N'-diméthyl-p-xylylène-diamine.

8. Cônes d'obturation du canal radiculaire dentaire suivant la revendication 1, dans lesquels ladite charge est un composé inorganique tel que La₂O₃, ZrO₂, BiPO₄, CaWO₄, BaWO₄, SrF₂, Bi₂O₃ ou des charges organiques, telles que des granules de polymère, des éclats de polymère ou une association de charges organiques et/ou inorganiques.

9. Cônes d'obturation du canal radiculaire dentaire suivant la revendication 1, contenant des charges qui confèrent une radio-opacité d'au moins 3 mm/mm de Al, avantageusement d'au moins 5 à 7 mm/mm de Al, de préférence d'au moins 7 mm/mm de Al.

10. Cônes d'obturation du canal radiculaire dentaire suivant la revendication 1, lesdits cônes étant solubles dans des solvants organiques polaires tels que CHCl₃, le tétrahydrofuranne ou le diméthylformamide, lesdits cônes pouvant être produits à partir de ces solutions de polymères.

11. Cônes d'obturation du canal radiculaire dentaire suivant la revendication 1, lesdits cônes contenant des additifs tels qu'un stabilisant et un plastifiant.

12. Procédé pour la préparation de cônes d'obturation du canal radiculaire dentaire de la revendication 1, comprenant : une charge et un polymère thermoplastique, dans lesquels ledit polymère thermoplastique est formé par polymérisation d'un monomère diépoxyde polymérisable et d'un monomère amine, lesdits monomères amine étant une monoamine primaire et/ou une diamine disecondaire, ladite charge représentant 40 à 90 % en poids desdits cônes conférant une radio-opacité d'au moins 3 mm/mm d'aluminium, **caractérisé par** une polymérisation, par addition thermique, du monomère diépoxyde et du monomère amine et un travail ou procédé thermique ultérieur.

13. Procédé suivant la revendication 12, comprenant les étapes :
i) de polymérisation, par addition thermique, du monomère diépoxyde et du monomère amine sur la surface de la charge ; et
ii) d'un procédé de formage (coulée) de la charge modifiée en surface de i) par des procédés thermiques et/ou sous pression.
